# EUROPEAN PATENT SPECIFICATION

(11) **EP 3 026 993 B1**
(45) Date of publication and mention of the grant of the patent: **22.08.2018**
(21) Application number: 15196374.1
(22) Date of filing: 25.11.2015
(51) Int. Cl.: H05K 9/00, A41D 31/00, A61N 1/16

(54) **SCREENING FABRIC FOR HIGH-FREQUENCY RADIO WAVES**
ABSCHIRMENDEN GEWEBE FÜR HOCHFREQUENZ-RADIOWELLEN
TISSU DE BLINDAGE POUR LES ONDES RADIO À HAUTE FRÉQUENCE

(30) Priority: 26.11.2014 IT AN20140183
(43) Date of publication of application: 01.06.2016
(73) Proprietor: Ingen S.r.l., 60035 Jesi (AN) (IT)
(72) Inventor: GENANGELI, Giorgio, 60035 Jesi (AN) (IT); GENANGELI, Michele, 60035 Jesi (AN) (IT)
(74) Representative: Baldi, Claudio

(56) References cited:
- WO-A1-2010/033093

## Description

The present patent application for industrial invention relates to **a production method of** a shielding fabric used to filter the high-frequency radio waves that pass through the same fabric.

It must be noted that the term "high-frequency radio waves" is used in the description and in the claims to indicate radio waves with frequency higher than 300 MHz.

This type of fabric has been developed in order to protect individuals from the high-frequency radio waves contained in the environment and generated by the electronic appliances that emit a radio or similar signal during operation, such as TVs, microwave ovens, tablets, smartphones, computers, Bluetooth devices, radio remote controls, Wi-Fi devices. etc.

The technological progress of the aforementioned electronic appliances and the increasingly large presence of these devices in our daily life have resulted in a macroscopic increase of radio waves.

Protections designed to shield the human body are already known for specific work environments. These products are generally made of several layers that are cumbersome, not versatile, rather uncomfortable and are often internally provided with materials that are harmful for the user.

WO2010033093 discloses a shielding fabric used to filter electromagnetic waves comprising polypropylene fibers and fibers coated with silver alloy containing 90% silver, 5% rhodium and 5% palladium. Rhodium compounds are very rare, extremely harmful and carcinogenic because they can cause severe skin damage that includes marking. Moreover, such a fabric is obtained with double jersey knitting, wherein fibers coated with silver alloy and polypropylene fibers are woven to form a net structure in such manner that all fibers of the fabric come in contact with the user's body. The fabrics obtained with double jersey knitting are generally characterized by high weight and poor versatility. Therefore, such a fabric is not suitable for making apparel items, it being very rigid and rather uncomfortable. Moreover, such a type of fabric is used to obtain a finished product made up of several layers of fabrics, which is impossible to use for apparel items.

The purpose of the present invention is to remedy the drawbacks of the prior art by providing **a production method of** a shielding fabric that is versatile, safe, comfortable and suitable for being used also in the apparel sector to make clothing items, either completely or partially.

Moreover, the purpose of the present invention is to disclose a **production method of** a shielding fabric that can be used to make underwear items or, more generally, items intended to come into direct contact with the user's skim, such as caps, hats, furnishing accessories, products for the building sector and the like.

The applicant has started from the consideration that a fabric comprising yarns made of conductive material should be suitable for shielding electromagnetic fields. Nevertheless, the use of conductive yarns results in poor hygienic characteristics and low comfort when in contact with the user's skin.

Therefore the applicant has decided that it was necessary to obtain a fabric comprising yarns of conductive material and yarns of non-conductive material that are suitably woven together. The yarns of conductive material consist in a core of synthetic material, such as polypropylene, polyamide and polyester and a coating of conductive metal material, such as copper, silver, or gold. Synthetic yarns, such as polypropylene, polyamide and polyester, and natural yarns, such as wool, cotton and hemp, have been used as non-conductive yarns.

Then the applicant has made a series of fabric samples obtained by knitting conductive yarns with non-conductive yarns. The fabric samples were different in terms of materials and type of knitting.

Several laboratory tests were made in order to define the performance of the fabric samples upon changing the characteristics of the electromagnetic field to be shielded and upon changing the type of yarn and weaving.

Electromagnetic interference with variable frequency was generated with a test generator, identifying a set of discreet frequencies within a high-frequency range from 300 MHz to 6 GHz, in order to evaluate the shielding properties of the fabric samples upon changing the yarns and the weave. The tests were carried out in a shielded anechoic room (without external electromagnetic phenomena) in such manner to guarantee measures and assessments with an adequate reproducibility level. The measurement methodologies are based on the CISPR, CEI and EN standards used in the EMC and safety sectors.

The following instruments were used for the tests:
- a generator of magnetic fields to generate, amplify and propagate electromagnetic fields; and
- a detector of magnetic fields to acquire and assess the electromagnetic fields generated by the generator.

The instruments were calibrated by generating sample magnetic fields with discreet frequencies comprised between 300 MHz and 6 GHz and saving the intensity of the sample electromagnetic fields detected by the detector without any interference.

After recording the calibration values of the sample electromagnetic fields, the tests were repeated by inserting the fabric samples between the generator and the receiver, generating the sample electromagnetic fields with the generator and receiving, through the receiver, the electromagnetic fields filtered by the fabric.

The values of the electromagnetic fields filtered by the sample fabrics were compared with the values of the calibration electromagnetic fields and it was discovered that the intensity of the electromagnetic fields filtered by the sample fabrics was lower than the intensity of the calibration electromagnetic fields.

Surprisingly, the test showed that not only the material, but also the shape of the weave significantly affected the shielding properties of the fabric. In fact, the wavelength of the electromagnetic interference to be shielded is strongly related with the weave of the material of the shielding fabric. Consequently, the best shielding results were obtained with fabrics woven in such a way to form a mesh with cells having lower dimensions than the wavelength to be shielded. The best results were obtained for cells with diameter lower than 3 mm.

Unexpectedly, the sample fabrics obtained by knitting two opposite yarns showed the best shielding results because the meshes of the two yarns overlapped. This type of knitting provides for weaving the two yarns in such a way that the conductive yarn is directed in one direction and the non-conductive yarn is directed in the opposite direction, in such manner to form a first side of the fabric made of conductive yarn only and a second side of the fabric made of non-conductive yarn only. Moreover, an additional advantage of such a type of fabric was the fact that the user's skin only came in contact with the side of the fabric made of non-conductive yarn.

According to the tests it was possible to select the fabric of the invention, which comprises a conductive yarn and a non-conductive yarn arranged in such manner that the conductive yarn is disposed in one direction and the non-conductive yarn is disposed in the opposite direction, thus forming a first side of the fabric made of conductive yarn only and intended to be faced outwards and a second side of the fabric made of non-conductive yarn only and intended to be faced towards the user's skin in order to obtain a more comfortable fabric.

According to a preferred embodiment, the weave was chosen in such a way to create a net with a plurality of eyelets or gaps having an internal diameter or an average dimension lower than 3 mm.

Preferably, the core of the conductive yarn is made of polyamide and the coating of the conductive yarn is made of silver. Preferably the non-conductive yarn is made of polypropylene.

After choosing the yarn of the invention, additional tests were made on the preferred embodiment of the fabric of the invention.

A transmitting antenna connected to the transmitter and a receiving antenna connected to the receiver and set to receive in the 30 MHz - 1 GHz range were positioned in the anechoic room.

Initially, the background noise of the instruments in the anechoic room was measured, without generating any signal. Then, leaving the space between the transmitting and the receiving antennas empty, a sinusoidal signal of -30 dBm level was applied to the transmitting antenna using the generator. The receiver connected to the receiving antenna was set for a CISPR-AVERAGE measure. The sinusoidal signal of -30 dBm level was repeated for discreet frequencies from 30 MHz to 1 GHz.

Successively, after discovering that the signal level received by the receiver was on average 50 dB higher than the background noise, the signal level was measured after positioning the fabric of the invention at 40 cm from the tip of the transmitting antenna.

The fabric of the invention has shown a shielding capacity that varies according to the frequency:
1) attenuation of about 1dB between 30 MHz and 90 MHz;
2) increasing attenuation from a minimum of 2.4 dB to 7.7 dB between 100 MHz and 600 MHz;
3) increasing attenuation from a minimum of 16.5 dB to 17.9 dB between 700 MHz and 1 GHz.

The tests showed that higher shielding is obtained upon increasing the frequency in the electromagnetic field. In particular, the maximum shielding value of approximately 16.5-17.9 dB was obtained for frequencies comprised between 700 MHz and 1 GHz, which are the typical frequencies of cellular phones, According to these results, because of its high shielding properties, the fabric of the invention can be used to make pockets of pants or jackets intended to contain electronic devices, such as cellular phones and smartphones, in order to protect the user's body from the electromagnetic waves emitted by said electronic devices.

More generally, the fabric **obtained with the method** of the invention can be used for underwear items, caps and T-shirts capable of protecting vital parts of the human body.

Moreover, the fabric **obtained with the method** of the invention can be customized according to the type of electromagnetic field from which the user needs to be protected, by changing the weave and the knitting of the two yarns.

For a better understanding the description of **the production method of** the fabric of the invention continues with reference to the attached drawings, which only have an illustrative, not limiting value, wherein:
Fig. 1 is a schematic view of the fabric according to a first embodiment;
Fig. 2 is an axial sectional view taken along the sectional plane II-II of Fig. 1;
Fig. 3 is a top view of the side facing towards the outside of the fabric according to a second embodiment;
Fig. 4 is a perspective view that shows the arrangement of the two yarns forming a multiple yarn used to obtain the fabric of Fig. 3;
Fig. 5 is a bottom view of the side facing towards the inside of the fabric according to the second embodiment; and
Fig. 6 is an enlarged sectional view taken along the sectional plane VI-VI of Fig. 3.

The fabric **obtained with the method** of the invention comprises a yarn made of conductive material (1) and a yarn made of non-conductive material (2).

The non-conductive yarn (2) can be a yarn of synthetic polymeric material, such as polypropylene. Polypropylene was chosen because it is a sufficiently elastic and soft yarn that is comfortable in contact with the user's skin.

The conductive yarn (1) comprises a core (10) made of a synthetic polymeric material, such as polyamide and a coating (11) made of an electric conductive material, such as silver. In addition to being sufficiently flexible and knittable, polyamide was chosen because it is the most appropriate synthetic material to support a silver coating. Silver was chosen as coating (11) because it is the best compromise between the cost and the conductive characteristics of the material and also for its anti-bacterial properties. In this case the silver coating (11) can be obtained with a galvanic bath.

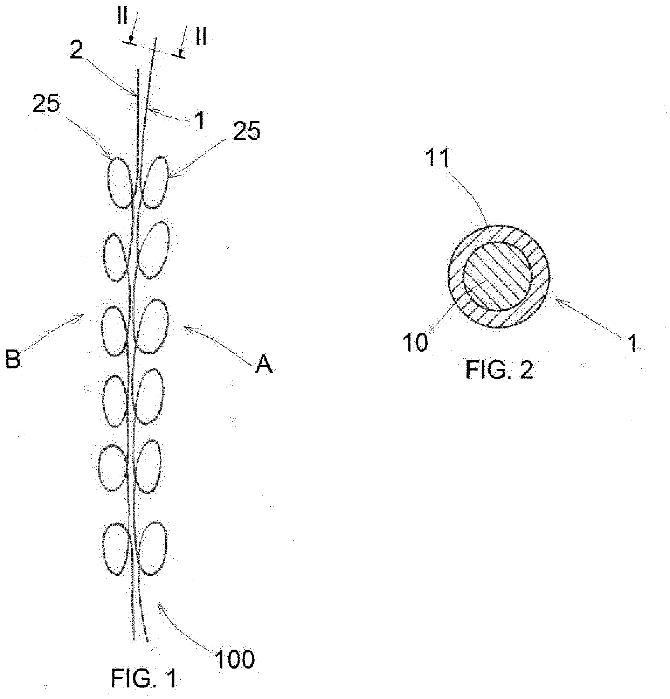

Fig. 1 shows a first embodiment of the fabric, which is generally indicated with numeral reference (100). In the fabric (100) of the invention the conductive yarn (1) and the non-conductive yarn (2) are woven in such manner that the conductive yarn (1) is directed in one direction and the non-conductive yarn (2) is directed in the opposite direction, in such manner to form a first side (A) of the fabric made of conductive yarn only and a second side (B) of the fabric made of non-conductive yarn only. In Fig. 1, the conductive yarn (1) is shown in bold to distinguish it from the non-conductive yarn (2).

As shown in Fig. 1, the conductive yarn (1) forms a plurality of eyelets (15) that are all faced towards a direction in order to form the first side (A) of the fabric. The conductive yarn (1) forms a plurality of eyelets (25) that are all faced towards a direction opposite to the first side (A) of the fabric, in order to form the second side (B) of the fabric.

The second side (B) of the fabric is intended to be directed towards the user's skin. Consequently, a clothing item made with the fabric of the invention is comfortable and not harmful for the user's health.

The fabric (100) of the first embodiment has a weave that comprises meshes consisting in the eyelets (15, 25) formed by the two yarns (1, 2). The eyelets (15, 25) have a substantially circular shape with a diameter lower than 3 mm. The meshes act as micro-cells that absorb and dissipate the electromagnetic waves because of the Faraday cage principle.

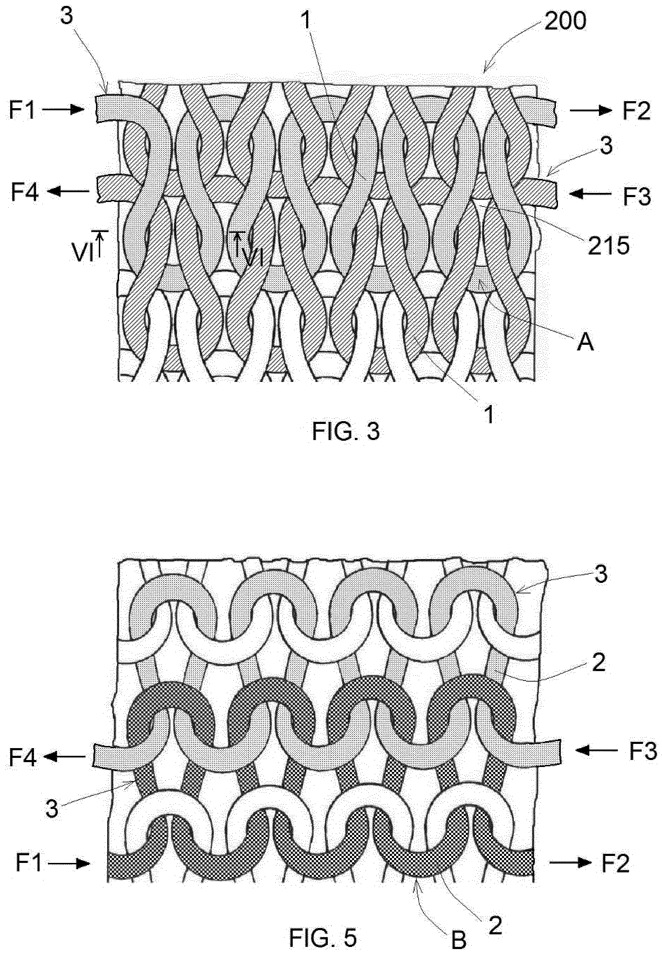

Referring to Figs. 3-6 a second embodiment of the fabric of the invention is described, which is generally indicated with numeral reference (200).

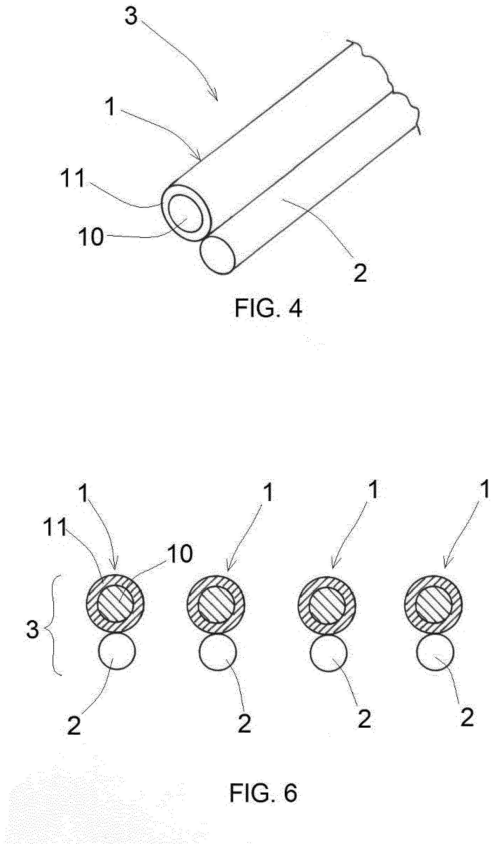

As shown in Fig. 4, the fabric (200) is obtained with a multiple yarn (3) that comprises the conductive yarn (1) and the non-conductive yarn (2) one next to the other.

As shown in Fig. 3, the multiple yarn (3) is fed in a weaving machine in such a way to follow a substantially sinusoidal path. A sinusoidal forward path, which is shown in bold, goes from arrow F1 to arrow F2. When the multiple yarn (3) arrives to the edge of the fabric, it is woven in the edge of the fabric in order not to come unsewn, and returns back following a sinusoidal backward path, which is shown as a broken line from F3 to F4. Along the backward path, the multiple yarn (3) is interconnected with the multiple yarn of the sinusoidal path, forming meshes similar the chains of a coat of mail.

During the sinusoidal forward and backward paths, the conductive yarn (1) is always maintained above the non-conductive yarn (2), as shown in the sectional view of Fig. 6. In fact, the plan view of Fig. 3 only shows the conductive yarn (1), and the non-conductive yarn (2) is not visible. Instead, the plan view of Fig. 5 only shows the non-conductive yarn (2), and the conductive yarn (1) is not visible.

Therefore, the fabric (200) has a first side (A) (Fig. 3) that is only made of the conductive yarn (1) and a second side (B) (Fig. 5) that is only made of the non-conductive yarn (2).

Advantageously, the weave of the meshes that are visible on the first side (A) (Fig. 3) is thicker than the weave of the meshes that are visible on the second side (B) (Fig. 5). For example, the meshes of the first side (A) of the fabric forms gaps (215) having a substantially elongated shape with average dimension lower than 3 mm. The term "average dimension" means the average between the higher dimension and the lower dimension. The gaps (215) act as micro-cells that absorb and dissipate the electromagnetic waves because of the Faraday cage principle.

The fabric of **obtained with the method of** the invention can be obtained with rectilinear weaving machines. According to the type of loom, an endless number of fabric types can be obtained using different yarns with a different size.

In the fabric (100, 200) according to the first or second embodiment, the yarns (1, 2) or the multiple yarn (3) create meshes that are much smaller than the wave length used by the majority of household electronic devices (Wi-Fi, cellular phones, computers, Bluetooth, wireless etc.). This allows the fabric (100, 200) to shield the delicate parts of the human body (heart, brain, genitals, organs and soft tissue in general), protecting them from the harmful effects of the waves.

## Claims

1. Production method of a shielding fabric (100; 200) used for filtering the high-frequency radio waves that pass through the fabric, wherein said fabric (100; 200) comprises a conductive yarn (1) and a non-conductive yarn (2) arranged in such manner that the conductive yarn (1) is disposed in one side of the fabric and the non-conductive yarn (2) is disposed in a opposite side of the fabric, thus forming a first side (A) of the fabric made of conductive yarn only and intended to be faced outwards and a second side (B) of the fabric made of non-conductive yarn only and intended to be faced towards the user's skin in order to obtain a more comfortable fabric,
**characterized in that** the production method comprises the following steps:
said fabric (2) is obtained with a multiple yarn (3) comprising said conductive yarn (1) placed side by side with said non-conductive yarn (2),
said multiple yarn (3) is fed in a weaving machine in such a way to follow a substantially sinusoidal path,
when the multiple yarn (3) arrives to an edge of the fabric, it is woven in the edge of the fabric in order not to come unsewn, and returns back following a sinusoidal backward path, so that said multiple yarn (3) travels along sinusoidal forward and backward paths,
along the backward path, the multiple yarn (3) is interconnected with the multiple yarn (3) of the sinusoidal path, forming meshes,
during the sinusoidal forward and backward paths, the conductive yarn (1) is always maintained overlapped to the non-conductive yarn (2), such a way that only the conductive yarn (1) is faced towards said first side (A) of the fabric and only the non-conductive yarn (2) is faced towards said second side (B) of the fabric.

2. The method of claim 1, wherein said conductive yarn (1) comprises a core (10) made of non-conductive material and a coating (11) made of conductive material.

3. The method of claim 2, wherein said core (10) is made of a synthetic polymeric material.

4. The method of any one of claims 2 to 3, wherein said coating (11) is of a metal material, such as silver.

5. The method of any one of the preceding claims, wherein said non-conductive yarn (2) is made of polypropylene.

6. The method of any one of the preceding claims, wherein said fabric (100; 200) has a weave comprising meshes with eyelets (15, 25) or gaps (115) having a substantially circular or elongated shape and a diameter or average dimension lower than 3 mm.

7. Clothing item comprising a shielding fabric obtained with the method according to one of claims 1 to 6.

8. Clothing item according to claim 7, wherein it is a underwear items.

9. Use of a shielding fabric obtained with the method according to any one of claims 1 to 6 as clothing item to shield the magnetic fields with frequency comprised between 300 MHz and 6 GHz.

## Patentansprüche

1. Verfahren zur Herstellung eines abschirmenden Gewebes (100; 200), das in der Lage ist, das Gewebe durchdringende hochfrequente Funkwellen abzuschirmen, wobei das Gewebe (100; 200) ein leitfähiges Garn (1) und ein nicht-leitfähiges Garn (2) umfasst, die derart angeordnet sind, dass das leitfähige Garn (1) auf einer Seite des Gewebes und das nicht-leitfähige Garn (2) auf der entgegengesetzten Seite des Gewebes angeordnet ist, derart, dass eine erste Seite (A) des Gewebes gebildet wird, die nur aus leitfähigem Garn besteht und dazu bestimmt ist, nach außen gerichtet zu werden, und eine zweite Seite (B) des Gewebes, die nur aus nicht-leitfähigem Garn besteht und dazu bestimmt ist, der Haut eines Trägers zugewandt zu werden, derart, dass das Gewebe bequemer wird,
**dadurch gekennzeichnet, dass** das Verfahren folgende Schritte umfasst:
das Gewebe (2) ist ausgehend von einem Mehrfachgarn (3) erhalten, welches das leitfähige Garn (1) neben dem nicht-leitfähigen Garn (2) umfasst,
das Mehrfachgarn (3) wird einer Webmaschine zugeführt, um im Wesentlichen sinusförmige Bahnen auszuführen,
wenn das Mehrfachgarn (3) an eine Kante des Gewebes gelangt, wird es in die Kante des Gewebes eingeflochten, um nicht auszufransen, und eine sinusförmigen Bahn ausführend zurückgeführt, derart, dass das Mehrfachgarn eine sinusförmige Vorwärts- und Rückwärtsbahn ausführt,
auf der Rückwärtsbahn wird das Mehrfachgarn (3) mit dem Mehrfachgarn (3) der sinusförmigen Vorwärtsbahn verkettet und bildet dadurch Maschen,
auf den sinusförmigen Vorwärts- und Rückwärtsbahnen ist das leitfähige Garn (1) stets zur ersten Seite des Gewebes gerichtet, während das nicht-leitfähige Garn (2) stets zur zweiten Seite des Gewebes gerichtet ist, über dem nicht-leitfähigen Garn (2), derart, dass nur das leitfähige Garn (1) zur ersten Seite (A) des Gewebes und nur das nicht-leitfähige Garn (2) zur zweiten Seite (B) des Gewebes gerichtet ist.

2. Verfahren nach Anspruch 1, wobei das leitfähige Garn (1) einen Kern (10) aus einem nicht-leitfähigen Material und eine Ummantelung (11) aus einem leitfähigen Material umfasst.

3. Verfahren nach Anspruch 2, wobei der Kern (10) aus einem synthetischen Material vom Polymertyp hergestellt ist.

4. Verfahren nach einem der Ansprüche 2 bis 3, wobei die Ummantelung (11) aus einem metallischen Material wie Silber hergestellt ist.

5. Verfahren nach einem der vorstehenden Ansprüche, wobei das leitfähige Garn (2) aus Polypropylen hergestellt ist.

6. Verfahren nach einem der vorstehenden Ansprüche, wobei das Gewebe (100; 200) eine Bindung aufweist, die Maschen mit Öffnungen (15, 25) oder Zwischenräumen (115) umfasst, die eine im Wesentlichen kreisrunde oder längliche Form und einen Durchmesser oder durchschnittliche Abmessungen von weniger als 3 mm aufweisen.

7. Kleidungsstück umfassend ein abschirmendes Gewebe, das mit dem Verfahren nach einem der Ansprüche 1 bis 6 erhalten ist.

8. Kleidungsstück nach Anspruch 7, wobei es sich um Unterwäsche handelt.

9. Verwendung eines abschirmenden Gewebes, das mit dem Verfahren nach einem der Ansprüche 1 bis 6 erhalten ist, als Kleidungsstück zur Abschirmung gegenüber Magnetfeldern mit Frequenzen zwischen 300 MHz und 6 GHz.

## Revendications

1. Méthode de protection d'un tissu de blindage (100; 200) en mesure de filtrer les ondes radio à haute fréquence qui traversent le tissu, où ledit tissu (100; 200) comprend in fil conducteur (1) et un fil non conducteur (2) disposés de manière que le fil conducteur (1) soit accommodé sur une face du tissu et le fil non conducteur (2) soit accommodé sur une face opposée du tissu, de manière à former une première face (A) du tissu composée uniquement par le fil conducteur destinée à être orientée vers l'extérieur et une seconde face (B) du tissu composée uniquement par le fil non conducteur destinée à être orientée vers la peau de l'utilisateur afin de rendre le tissu plus confortable,
**caractérisé en ce que** la méthode de production comprend les phases suivantes :
ledit tissu (2) est obtenu à partir d'un fil multiple (3) comprenant dit fil conducteur (1) juxtaposé au dit fil non conducteur (2),
ledit fil multiple (3) est alimenté dans une machine à tisser de façon à effectuer des parcours essentiellement sinusoïdaux,
lorsque le fil multiple (3) atteint un bord du tissu, il est tressé dans le bord du tissu, pour ne pas se défiler, et il retourne en arrière en effectuant un parcours sinusoïdal de retour, de façon que le fil multiple effectue un parcours sinusoïdal d'aller-retour,
pendant le parcours de retour, le fil multiple (3) s'enchaîne avec le fil multiple (3) du parcours sinusoïdal de l'aller, en formant des mailles,
pendant les parcours sinusoïdaux d'aller et retour, le fil conducteur (1) se maintient toujours orienté vers la première face du tissu et le fil non conducteur (2) se maintient toujours orienté vers la seconde face du tissu, au-dessus du fil non conducteur (2), de façon que seul le fil conducteur (1) soit disposé vers ladite première face (A) du tissu et seul le fil non conducteur (2) soit orienté vers ladite seconde face (B) du tissu.

2. Méthode selon la revendication 1, où ledit fil conducteur (1) comprend une âme (10) en matériel non conducteur et un revêtement (11) en matériel conducteur.

3. Méthode selon la revendication 2, où ladite âme (10) est réalisée en matériel synthétique de type polymérique.

4. Méthode selon l'une quelconque des revendications de 2 à 3, où ledit revêtement (11) est en matériel métallique, comme l'argent.

5. Méthode selon l'une quelconque des revendications précédentes, où ledit fil conducteur (2) est en polypropylène.

6. Méthode selon l'une quelconque des revendications précédentes, où ledit tissu (100; 200) présente une trame comprenant des mailles ayant des oeillets (15, 25) ou des interstices (115) ayant une forme essentiellement circulaire ou allongée et un diamètre, ou dimension, moyen inférieur à 3 mm.

7. Vêtement comprenant un tissu de blindage obtenu avec la méthode selon l'une des revendications de 1 à 6.

8. Vêtement selon la revendication 7, où le vêtement est un sous-vêtement.

9. Utilisation d'un tissu de blindage obtenu avec la méthode selon l'une quelconque des revendications de 1 à 6, en tant que vêtement pour blinder les champs magnétiques ayant une fréquence comprise entre 300 MHz et 6 GHz.
